# EUROPEAN PATENT APPLICATION

(11) **EP 2 859 947 A1**
(43) Date of publication of application: **15.04.2015**
(21) Application number: 13188450.4
(22) Date of filing: 14.10.2013
(51) Int. Cl.: B01L 3/00, C12Q 1/68, G01N 33/487

(54) **Electrical polynucleotide mapping**

(71) Applicant: IMEC VZW, 3001 Leuven (BE); Katholieke Universiteit Leuven, 3000 Leuven (BE)
(72) Inventor: Stakenborg, Tim, 3001 Leuven (BE); Neely, Robert, 3001 Leuven (BE); Van Dorpe, Pol, 3001 Leuven (BE); Hofkens, Johan, 3001 Leuven (BE)
(74) Representative: Ego, Christophe

(57) **Abstract**

A micro-fluidic device (100) for mapping a DNA or RNA strand (105) labeled at a plurality of specific sites with labels (106) suitable for generating a detection signal when interacting with a detector element, the device comprising:
- a micro-fluidic channel (101); and
- a plurality of detector elements (102, 103) for detecting the labels (106) by acquiring the detection signals, the detector elements (102, 103) being positioned longitudinally along the micro-fluidic channel (101), each detector element (102, 103) having a width, successive detector elements (102, 103) being separated by an inter-detector gap having a width, wherein the widths of at least two of the detector elements (102, 103) are different and/or wherein the widths at least two of the inter-detector gaps are different.

## Description

### Technical field of the invention

The present invention relates to the field of polynucleotide mapping, and more specifically to a micro-fluidic device for mapping a polynucleotide such as a DNA or RNA strand.

### Background of the invention

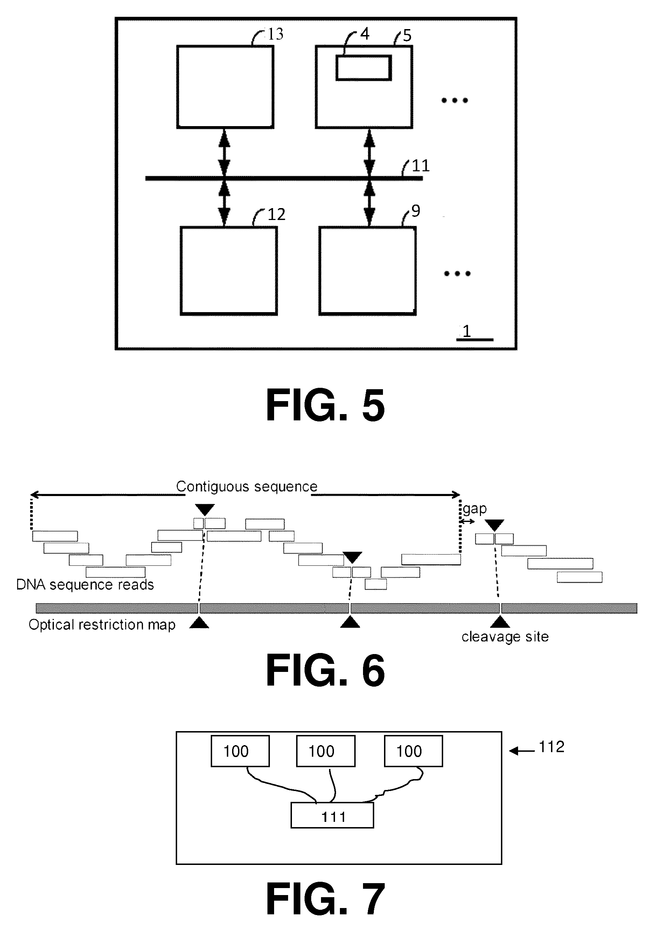

The progress in DNA sequencing technologies has provided us with unprecedented insight in the understanding of our genome. Sequencing a DNA molecule has never been easier or faster using the new generation of DNA sequencing technologies. The speed of reading the sequence of an entire genome as well as it costs is rapidly dropping. Whilst there have been some spectacular scientific and technological developments in this field, it is widely accepted that the next great barrier to progress in the field of genomics will not be the sequencing technology but our ability to handle the incredible amount of data and trace it back to clinical valuable data. Central to the issue of the computational workload imposed by sequencing technologies of the last generation is the fact that the average read length of single molecule sequencing platforms is typically much less than 400 base pairs; far shorter than the read lengths generated by the earlier Sanger sequencing based platforms. This largely complicates genome assembly, hence, introducing again a burden on the computational power needed to handle the data. The implications of short read length were highlighted in the de novo sequencing of the giant panda genome using a current (Illumina) sequencing methodology. Using short sequences of 50-75 nucleotides in length and an average 96 fold coverage, the final draft genome covered only about 93.8% of the genome. This can be compared with the similarly-sized dog genome, which was sequenced de novo using Sanger sequencing (typical read lengths of around 800 bases) and only a 7.5-fold coverage, for which the draft genome covered nearly 99% of the genome. Also the N50 contig length was close to 180 kb for the dog genome, while only 37 kb for the panda genome. The number of gaps in an assembled genome is critical, since with each gap, the opportunity to study large scale structural variations in the genome diminishes. In fact, genomic structural variations, which include duplications, deletions, insertions, inversions and translocations of large genomic regions (typically from 1 kb to several Mbases in length) are clinically relevant to detect. Indeed, structural variations in the human genome have been found to influence at least the function of 3863 genes (12.5% of the genes from the human genome as described in the NCBI RefSeq database). The abundance of large-scale structural variations in the human genome and their significance, from a phenotypic perspective, make these variations important targets for future genomic studies and this underscores the necessity for their inclusion in the final, validated versions of genomic sequences. To facilitate the assembly of a genome sequence which allows the incorporation of both relatively short as well as long structural variations, a DNA mapping technology is useful. In this technology, DNA is labeled at specific sites and the order and position of these sites is visualized generating a quick overview of the entire sequence. As such, an optical DNA map or DNA barcode provides a context without giving the full detailed individual sequence of single nucleotides. As DNA maps are preferably made for large DNA fragments, significant genomic regions can be spanned giving also a means to study larger structural variations that is difficult to assess with current sequencing technologies. Such a technology permits to obtain a map which can serve as a scaffold for the assembly of the genome sequence. Fig. 6 illustrates how this is performed with an optical restriction map according to the prior art. The map gives information on the distance between sites separated by several kilobases, which can be used in order to define the size and location of gaps in the genomic assembly. The map serves as a scaffold for the genomic assembly, allowing the correct positioning and orientation of contiguous sequences.

Sequencing of whole genomes remains too challenging to be performed routinely for clinical applications. For many applications, not the entire sequence is needed and a general overview of the genome, possibly showing structural variations, is sufficient. DNA mapping can provide such data. In several applications that utilize genomic analysis, including for example the rapid identification of pathogens, a complete genomic sequence is not a requisite. In fact, all that is needed is a quick overview of the genome structure and sequence, which can be provided by DNA mapping or DNA barcoding. Another important application of optical mapping is for instance the rapid identification and characterization of bacterial species and strains. Naturally, many other applications in need of identification or comparative genomics can be thought of. In such instances, it is often important to do this as rapidly and with as low a cost as possible. Hence, for such applications, DNA mapping is normally intrinsically more cost-effective compared to sequencing. Complementary information is derived from sequencing and optical mapping.

Current mapping technologies using nanochannels use standard optical methods to visualize fluorescently labeled DNA.

Nanofluidic devices with channels that have dimensions that approach the persistence length of the DNA (∼50nm) can be used to linearize DNA molecules in the solution phase.

The overarching aim of an optical map (normally for DNA, although almost the same reasoning could hold for RNA maps) is to provide a sequence specific barcode for a genomic fragment or for a genome. To this end, optical labels, typically fluorophores, are introduced using a sequence specific labeling method and the distance between such labels is recorded.

Although sub-diffraction-limit imaging techniques have been described, in general, techniques using optical labels suffer from a relatively low resolution of 250-300 nm due to the diffraction limit associated with optical microscopic methods. When considered in terms of length along a DNA molecule, this equates to a resolution of around 750 base pairs. Hence, the maximum achievable resolution of optical mapping techniques employing standard optical microscopy, is limited to approximately the length of a gene. The diffraction limit represents a fundamental barrier to the resolution achievable in DNA mapping using optical methods. In addition, the high-end single molecule optical techniques are typically expensive and bulky.

Also the throughput of DNA mapping is still limited in known set-ups. There are several flavors of optical DNA mapping platforms but all rely on the sequence-specific modification (either cleavage or fluorescent labeling) of DNA at short target sites, typically of 8 bases or less in length. The shorter the recognition sequence, the shorter the inter-distance between two subsequent labels. This is followed by imaging and analysis of the modified DNA such that the distances between cut sites or fluorophores is determined.

Naturally, a good labeling method is advantageous to the technique as the labeling directly corresponds to the quality and reproducibility of the genomic barcode. For optical DNA mapping, at least three enzymatic techniques have been described, employing DNA restriction enzymes, DNA nicking enzymes, or DNA methyltransferases. DNA can also be sequence-specifically labeled using triple-helix forming bis-PNA (peptide nucleic acid) compounds. Each strategy for DNA modification allows or requires a distinct experimental approach to enable the generation of an optical map. Amongst the established methods, optical restriction mapping is the most established of the single-molecule DNA mapping platforms. The optical map simply contains information about the relative positions of restriction enzyme sequences, typically chosen to be 6 or 8 bases in length. This technique enables the accurate sizing of fragments as small as ∼800 bases.

An alternative approach to restriction enzyme-based mapping is to employ an endonucleaze nicking enzyme to label the DNA with fluorophores, rather than to cut it. This can be done with high sequence-specificity. Using standard, diffraction-limited, fluorescence microscopy fluorophores can be resolved at distances on the order of 250 nm (800 bp). The great advantage of nick-based labeling is the highly sequence-specific labeling via a covalent bond of the fluorescent dye molecules to the DNA duplex.

Another method to create DNA map employs DNA methyltransferase enzymes for DNA labeling. The possibility to integrate optical labels using this method has recently been shown to sequence specifically (5'-GCGC-3') labeled bacteriophage lambda using methyltransferase M.HhaI. In this method, a DNA methyltransferase enzyme is used to direct the labeling to short sequences, typically of four or six bases in length. The methyltransferase is simply incubated along with the DNA and a synthetically prepared cofactor for this enzyme. In the native reaction, the methyltransferase catalyses the transfer of a methyl group from the cofactor s-adenosyl-L-methionine (SAM) to either a cytosine or adenine base, depending on the methyltransferase. Alternatively, other means to incorporate functional endgroups on DNA are possible (e.g. PNA DNA binders).

US 2010/0267158 discloses electronic detectors inside nanofluidic channels for detection, analysis, and manipulation of DNA molecules in the absence of any sequence-specific modification. This prior art contemplates that this device may be further developed into the next-generation DNA sequencer.

US 2005/0019784 discloses apparatus and methods relating to the sequencing and/or identification of nucleic acids. Disclosed are devices comprising nanopores operably coupled to detectors that can detect labeled nucleotides passing therethrough. The detection may occur by either photodetection or electrical detection. Where electrical detection is used, the electrical detector may detect any type of electrical signal and the nucleotides may be tagged with a label that can be detected by its electrical properties. Gold particles are cited for this purpose. The time interval between electrical signals may be measured and used to create a distance map representing the positions of labeled nucleotides on the nucleic acid molecule. An advantage of this approach is that the resolution can in principle be higher than in the case of optical detection. This is due to the fact that electrical detection does not suffer from the diffraction limit of optical methods. However, timing of the label detection is sub-optimal.

There is therefore a need in the art for a novel methodology to map nucleotides such as DNA and RNA which would overcome the above stated drawbacks.

### Summary of the invention

It is an object of the present invention to provide good apparatus or methods for mapping a DNA or RNA strand.

It is an advantage of embodiments of the present invention that a rapid genomic barcoding technology is provided, assuring high throughput.

It is an advantage of embodiments of the present invention that integrated devices can be provided. Embodiments of the present invention provide devices that permit the mapping of a DNA or RNA strand within a single compact device, in contrast to optical DNA mapping methods which require bulky optical reading means.

It is an advantage of embodiments of the present invention that a mapping of a DNA or RNA strand can be achieved with a high resolution.

The above objective is accomplished by a method and device according to the present invention.

In a first aspect, the present invention relates to a micro-fluidic device for mapping a DNA or RNA strand labelled at a plurality of specific sites with labels suitable for generating a detection signal when interacting with a detector element, the device comprising:
- a micro-fluidic channel ; and
- a plurality of detector elements for detecting the labels by acquiring the detection signals, the detector elements being positioned longitudinally along the micro-fluidic channel, each detector element having a width, successive detector elements being separated by an inter-detector gap having a width, wherein the widths of at least two of the detector elements are different and/or wherein the widths at least two of the inter-detector gaps are different.

In another aspect, the present invention relates to the use of the device according to any embodiment of the first aspect for performing the mapping of a DNA or RNA strand.

In a further aspect, the present invention relates to a method for mapping a DNA or RNA strand using a micro-fluidic device for mapping a DNA or RNA strand labeled at a plurality of specific sites with labels suitable for generating a detection signal when interacting with a detector element, the device being according to any embodiment of the first aspect, the device comprising at least
- a micro-fluidic channel; and
- a plurality of detector elements for detecting the labels by acquiring the detection signals, the detector elements being positioned longitudinally along the micro-fluidic channel, each detector element having a width, successive detector elements being separated by a inter-detector gap having a width, wherein the widths of at least two successive detector elements are different and/or wherein the width at least two of the inter-detector gaps are different;

The device being connected to, or comprising, a measurement unit connected to the plurality of detector elements, the measurement unit being adapted for receiving and for timing the detection signals from each of the detector elements and optionally for measuring the magnitude of the detection signals from each of the detector elements;

The device being connected to, or comprising, a means for moving the DNA or RNA strand through the micro-fluidic channel, the means being connected to the micro-fluidic channel.

This method comprises the steps of:
- providing a liquid comprising a DNA or RNA strand in the micro-fluidic channel, the strand being labeled at a plurality of specific sites by labels suitable for generating a detection signal when interacting with a detector element;
- moving the strand through the micro-fluidic channel;
- detecting the labels using the measurement unit and recording timing information of the detected labels;
- mapping the DNA or RNA strand by using the timing information of the detected labels for determining the distance between pairs of labels, thereby determining the distance between the specific sites.

In a further aspect, the present invention relates to a micro-fluidic chip comprising:
- a plurality of micro-fluidic devices according to any embodiment of the corresponding aspect of the present invention;
- a liquid supply unit connected to the plurality of micro-fluidic channels and arranged to provide a liquid comprising a plurality of DNA or RNA strands, each strand being labeled at a plurality of specific sites by labels suitable for generating a detection signal when interacting with a detector element, to the plurality of micro-fluidic channels. This aspect is advantageous as it permit a parallel format ensuring mapping with high speed.

In yet a further aspect, the present invention relates to a method for performing multiplexed mapping of DNA or RNA strands using the micro-fluidic chip as described above, making use of a plurality of micro-fluidic devices for mapping a DNA or RNA strand labeled at a plurality of specific sites with labels suitable for generating a detection signal when interacting with a detector element, each device comprising:
- a micro-fluidic channel;
- a plurality of detector elements for detecting the labels by acquiring the detection signals, the detector elements being positioned longitudinally along the micro-fluidic channel, each detector element having a width, successive detector elements being separated by a inter-detector gap having a width, wherein the widths of at least two successive detector elements are different and/or wherein the width at least two of the inter-detector gaps are different;
- a measurement unit connected to the plurality of detector elements, the measurement unit being adapted for receiving and for timing the detection signals from each of the detector elements and optionally for measuring the magnitude of the detection signals from each of the detector elements;
- a means for moving the DNA or RNA strand through the micro-fluidic channel, the means being connected to the micro-fluidic channel. The method comprises:
- providing a liquid comprising a plurality of DNA or RNA strands, each being labeled at a plurality of specific sites by labels suitable for generating a detection signal when interacting with a detector element, to the fluid supply unit;
- moving each of the plurality of DNA or RNA strands through any of the plurality of micro-fluidic channels using one or more means for moving the DNA or RNA strands;
- detecting the labels of each of the plurality of DNA or RNA strands using the measurement units;
- mapping each of the plurality of DNA or RNA strands using the computational unit by determining the distance between pairs of labels on each of the plurality of DNA or RNA strands using the timing information of each of the detected labels.

In a further aspect, the present invention also includes a computer program or computer program product which, when executed on computing means, provides instructions for executing any of the methods of the present invention.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a schematic representation of a vertical cross-section of device with an ionic read-out according to an embodiment of the present invention.
Fig. 2 is a schematic representation of a horizontal cross-section of a device with an impedimetric or capacitive read-out according to an embodiment of the present invention.
Fig. 3 is a schematic representation of a vertical cross-section of a device with a double layer capacitive read-out according to an embodiment of the present invention.
Fig. 4 is a schematic representation of a detection method according to an embodiment of the present invention.
Fig. 5 shows a processing system including the instructions to implement aspects of the methods according to embodiments of the present invention.
Fig. 6 shows an optical restriction map from the prior art.
Fig. 7 schematically represents a micro-fluidic chip according to an embodiment of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled" should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the invention, the invention being limited only by the terms of the appended claims.

Reference will be made to transistors. These are three-terminal devices having a first main electrode such as a drain, a second main electrode such as a source and a control electrode such as a gate for controlling the flow of electrical charges between the first and second main electrodes.

In a first aspect, the present invention relates to a micro-fluidic device for mapping a DNA or RNA strand labeled at a plurality of specific sites with labels suitable for generating a detection signal when interacting with a detector element.

Micro-fluidic devices are devices for the manipulation of fluids that are geometrically constrained to a sub-millimeter scale. The micro-fluidic devices of the present invention comprise a micro-fluidic channel and a plurality of detector elements.

The micro-fluidic channel is a channel, suitable for transporting a liquid, which smaller dimension is smaller than one millimeter. Preferably, its smaller dimension is smaller than one micron. In this case, the micro-fluidic channel is a nano-fluidic channel and the device comprising the nano-fluidic channel is a nano-fluidic device, i.e. a device for the manipulation of fluids that are geometrically constrained to a sub-micrometer scale. More preferably, the micro-fluidic channel has its smaller dimension smaller than 100 nm. Also, the micro-fluidic channel has its smaller dimension larger than 5 nm. For instance, the nano-fluidic channel has its smaller dimension comprised between 30 and 70 nm. Such dimensions are advantageous because the persistence length of a DNA strand is about 50 nm and such dimensions therefore permit to linearize the DNA molecules in the solution phase. Micro-fluidic channels can be manufactured by lithography. Micro-fluidic channels are advantageous as they permit to isolate the DNA or RNA strand from its surrounding. Another advantage of micro-fluidic channels is that they permit to linearize the strand. Depending on the buffer conditions and the channel dimensions, DNA can be extended up to around 60 to 70% of its maximal length as determined by crystallography. The use of micro-fluidic channels and more particularly of nano-fluidic channels permits to ensure single-molecule sensitivity.

The micro-fluidic channel can be manufactured for instance by lithography of a Complementary Metal-Oxide-Semiconductor (CMOS) substrate, which surface has been passivated.

The detector elements are suitable for detecting the labels by acquiring the detection signals. The detector elements are positioned longitudinally along the micro-fluidic channel, each detector element having a width, successive detector elements being separated by a inter-detector gap having a width.

Typically, the widths of at least two of the detector elements are different and/or the width at least two of the inter-detector gaps are different.

In embodiments, the plurality of detector elements is a plurality of detector elements of increasing width along the micro-fluidic channel.

In embodiments, the successive inter-detector gaps are of increasing width along the micro-fluidic channel.

In embodiments, the width of any detector element having two neighboring detector elements is larger than the width of one of the two neighboring detector elements and smaller than the width of the other of the two neighboring detector elements. In other words, the device may comprise at least three successive detector elements of increasing width along the micro-fluidic channel.

In embodiments, any of the inter-detector gaps surrounded by a preceding inter-detector gap and a following inter-detector gap has a width larger than the width of the preceding inter-detector gap and smaller than the width of the following inter-detector gap. In other words, the device may comprise at least three successive inter-detector gaps of increasing width along the micro-fluidic channel.

In embodiments, the width of any detector element having two neighboring detector elements is larger than the width of one of the two neighboring detector elements and smaller than the width of the other of the two neighboring detector elements and any of the inter-detector gaps surrounded by a preceding inter-detector gap and a following inter-detector gap has a width larger than the width of the preceding inter-detector gap and smaller than the width of the following inter-detector gap. In other words, the device may comprise at least three successive detector elements of increasing width along the micro-fluidic channel and/or at least three successive inter-detector gaps of increasing width along the micro-fluidic channel.

The width of a detector element can for instance be from 50 to 200 nm. The increment between successive detector elements can for instance be from 5 to 30 nm. This increment can be constant along the length of the microchannel or it can vary.

The width of an inter-detector gap can for instance be from 50 to 200 nm. The increment between successive inter-detector gaps can for instance be from 5 to 30 nm. This increment can be constant along the length of the microchannel or it can vary.

The detector element is typically suitable for electrically detecting the label. The detector element is typically a conductor-based detector element. For instance, it can be a transistor-based or an electrode based detector element. The structure of the detector element depends on the envisioned read-out scheme.

In an embodiment, each detector element may comprise two electrodes positioned longitudinally along the micro-fluidic channel, wherein both electrodes together span the width of the detector element; and wherein the detection signals are voltage changes between the two electrodes. This embodiment is suitable for instance for an ionic read-out scheme. In this embodiment, the micro-fluidic device may therefore further comprise a means for applying an ionic current through an electrolyte in the micro-fluidic channel.

In other embodiments, each detector element may comprise two electrodes facing each other on opposite sides of the micro-fluidic channel, each detector element being adapted for allowing the application of a DC bias superimposed to an AC voltage between the two electrodes and wherein the detection signals are impedance or capacitance changes. This embodiment is suitable for instance for an impedance or a capacitance based read-out scheme. The impedance is preferably measured at the AC frequency. The AC frequency is preferably selected in such a way as to maximize the label-induced signal. This can easily be achieved experimentally by trial and error. The optimal frequency can also be evaluated from the permittivity of both the label and the environment. In order to sense the particles, it may be better to increase the frequency to above the cut-off frequency of the ions in the solution, such that the measured capacitance is not influenced by the ion diffusion capacitance.

In yet other embodiments, the plurality of detector elements may be composed of:
a) a corresponding number of single electrodes, and
b) a single counter electrode common to each of the single electrodes, and the detection signal may be a double layer capacitance change .

This embodiment is suitable for instance for a double layer capacitance change based read-out.

As used herein and unless provided otherwise, mapping of a DNA or RNA strand refers to the determination of the distances between successive specific sites along a DNA or RNA strand. In embodiments where more than one type of specific site (e.g. more than one sequence of nucleotides) is labeled, mapping may refer to the determination of both the order of the specific sites and the distances between successive specific sites. Mapping in the sense of the present invention can also be referred to as barcoding. The term mapping is also used in the art to refer to the assignment of DNA fragments to chromosomes but this is not the sense in which the term mapping in used here. In embodiments, this determination of the distances between successive specific sites and optionally of the order of the specific sites along a DNA or RNA strand can be accompanied by a representation, e.g. a visual representation of the distances between successive sites and optionally their order. In embodiments where the order of the specific sites can be determined as well by the mapping, more than one type of label is typically used. For instance, a first type of specific site may be labeled with a first type of label suitable for generating a first type of detection signal (e.g. a voltage raise) while a second type of specific site may be labeled with a second type of label suitable for generating a second type of detection signal (e.g. voltage drop or a voltage raise of a different amplitude). As an example, a conductive label and a dielectric label could be used.

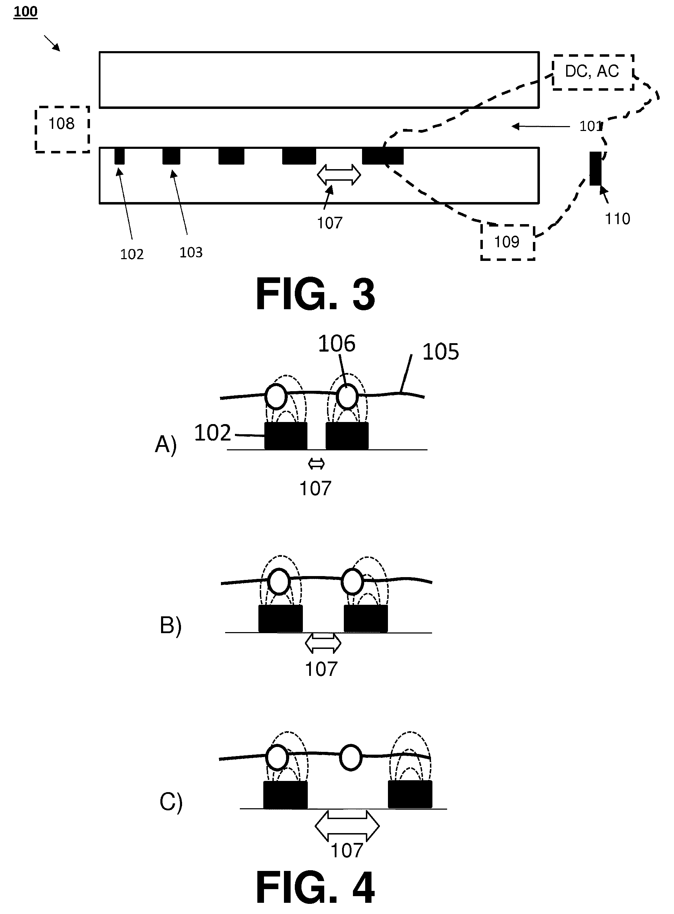

The density of the mapping achievable in embodiment of the present invention can be lower than one label per 50 nm, lower than one label per 20nm, lower than one label per 10 nm, lower than one label per 8 nm or even lower than one label per 7 nm. This resolution is mostly limited by the density of labels that can be introduced on the DNA or RNA strands since each label can be as small as e.g. 2 nm and the detection methods according to embodiments of the present invention (see especially the embodiments corresponding to examples 1-3) permit a spatial resolution of the detection as small as 1 nm. The embodiment of figure 3 has a slightly worse resolution of from 2 to 5 nm (limited by the resolution of the lithographic procedure used to produce the detectors themselves).

This high resolution permit to map DNA or RNA strands bearing a high density of labels.

As used herein and unless provided otherwise, labeling comprises attaching a label at a specific site of a DNA or RNA strand. In embodiments, the specific site can be a particular sequence of nucleotides. The labeling can therefore be sequence-specific. In embodiments, enzymes may be used to label the DNA or RNA strand. In a preferred embodiment, methyltransferases (see below) are used to label the DNA or RNA strand. This is advantageous as the linker between the polynucleoide specific site and the label can be made fairly short, thereby enabling a higher labeling density and a higher mapping resolution. However, the present invention is not necessarily limited to the use of methyltransferases and other labeling technologies known to the person skilled in the art can be used as well. For instance, Peptide Nucleic Acid (PNA) binders can be used as an alternative to the use of methyltransferases.

As used herein and unless provided otherwise, the labels are entities that can be attached to a specific site of a DNA or RNA strand and which is suitable for generating a detection signal when interacting with a detector element. In particular, the labels may be suitable for generating a detection signal (e.g. an electrical detection signal) when interacting with an electrical detector element.

The choice of the label (also called tag) depends on the type of detection scheme. If voltage, impedance or capacitance change is measured, the label is preferably metallic or dielectric. An example of metallic label is a metal nanoparticle such as a gold, a silver, a copper or an iron nanoparticle. An example of dielectric particle is a nanoparticle made of a dielectric material selected from silica, ZnSe, CdS, polystyrene, polymethylmethacrylate (PMMA), and Fe₃O₄. If double layer capacitance change is measured, the label is preferably a charged particle. It can however also be a metallic, dielectric or magnetic particle. Examples of charged particles are polystyrene particles having phosphate or sulfate groups on their surface. The term "electrical label" or "electrical tag" can be used to designate a label suitable for generation an electrical detection signal by interacting electrically with a detector element. Typically, such electrical labels are most suitable for use in the present invention.

The label is preferably a particle with very small dimensions in order not to decrease the resolution of the mapping. For instance, a nanoparticle or a molecular group can be used. Preferably, the nanoparticle has dimensions ranging from 1 to 50 nm, more preferably from 1 to 20 nm, yet more preferably from 1 to 10 nm and most preferably from 1 to 5 nm. In embodiments, the nanoparticle may have dimensions ranging from 2 to 50 nm.

The labels preferably have a dispersity from 1 to 1.5, preferably from 1 to 1.3, more preferably from 1.0 to 1.1. The label is preferably monodisperse. This increases the achievable resolution.

In an embodiment, the micro-fluidic device may further comprise a measurement unit connected to the plurality of detector elements, the measurement unit being adapted for receiving and for timing the detection signals from each of the detector elements and optionally for measuring the magnitude of the detection signals from each of the detector elements. Although the measurement unit is necessary for the performance of the mapping, the device can be a module separated from the measurement unit. In an embodiment, the device can comprise the measurement unit or can be connected to an external measurement unit. Preferably, in order to permit higher speed and/or multiplex capabilities, the measurement unit is preferably comprised in the device. For instance, the measurement unit can be an integrated CMOS electronic circuit. Preferably, the measurement unit is able to receive electronic signals. Preferably, the measurement unit is able to time electronic signals. Preferably, the measurement unit is able to send electronic signals.

In an embodiment, the micro-fluidic device may further comprise a means for moving the DNA or RNA strand through the micro-fluidic channel, the means being connected to the micro-fluidic channel. Examples of said means are a pressure difference between the inlet and outlet of the channel, a DC electrical field (electrophoresis), and a magnetic or optical trap pulling on a magnetic or dielectric bead attached at one end of the DNA or RNA strand.

In an embodiment, the device may further comprise means for linearizing a DNA or RNA strand, said means being fluidly connected to the channel.

In an embodiment, the device may further comprise a liquid supply unit connected to the micro-fluidic channel and arranged to provide a liquid comprising a DNA or RNA strand.

In another aspect, the present invention relates to the use of the device according to any embodiment of the first aspect for performing the mapping of a DNA or RNA strand.

The obtained map can be read and analyzed like a barcode.

In an embodiment, the mapping may be performed to acquire information about a genome.

In an embodiment, the mapping may be performed to identify a living organism such as a pathogen. As used herein, the term living organism includes any organism comprising DNA or RNA, including viruses. This is advantageous because a mapping as described herein can be performed rapidly compared to the performance of a complete genomic sequence, thereby enabling a rapid identification of the organism (e.g. pathogen).

In an embodiment, the mapping may be performed to identify the degree of relatedness of two distinct organisms.

In an embodiment, the mapping may be performed to acquire information about an epigenome. For this purpose, an enzyme for the labeling may be chosen in such a way that it is blocked if any one DNA base it targets is methylated. In an alternative embodiment, the labels used may be coupled to methylcytosine specific antibodies or proteins containing a methyl-CpG-binding domain.

Even if the full underlying genome is known (which is largely static within an individual), the epigenome can be dynamically altered by environmental conditions. Examples of such epigenetic changes are DNA methylation and histone modification, both of which serve to regulate gene expression without altering the underlying DNA sequence. Understanding how these markers vary among cell types will provide further info to understand how DNA is utilized and can control traits.

Current epigenomic analyses employ bisulfite sequencing and chromatin immunoprecipitation, but are still rather expensive, cumbersome to perform and require substantial input material.

In a further aspect, the present invention relates to a method for mapping a DNA or RNA strand using a micro-fluidic device for mapping a DNA or RNA strand labeled at a plurality of specific sites with labels suitable for generating a detection signal when interacting with a detector element, the device being according to any embodiment of the first aspect, the device comprising at least
- a micro-fluidic channel; and
- a plurality of detector elements for detecting the labels by acquiring the detection signals, the detector elements being positioned longitudinally along the micro-fluidic channel, each detector element having a width, successive detector elements being separated by a inter-detector gap having a width, wherein the widths of at least two successive detector elements are different and/or wherein the width at least two of the inter-detector gaps are different.

The device being connected to, or comprising, a measurement unit connected to the plurality of detector elements, the measurement unit being adapted for receiving and for timing the detection signals from each of the detector elements and optionally for measuring the magnitude of the detection signals from each of the detector elements.

The device being connected to, or comprising, a means for moving the DNA or RNA strand through the micro-fluidic channel, the means being connected to the micro-fluidic channel.

This method comprises the steps of:
- providing a liquid comprising a DNA or RNA strand in the micro-fluidic channel, the strand being labeled at a plurality of specific sites by labels suitable for generating a detection signal when interacting with a detector element;
- moving the strand through the micro-fluidic channel;
- detecting the labels using the measurement unit and recording timing information of the detected labels;
- mapping the DNA or RNA strand by using the timing information of the detected labels for determining the distance between pairs of labels, thereby determining the distance between the specific sites.

The method may further comprise a step of representing the distances between successive sites and optionally their order, e.g. via a visual representation.

The step of moving the strand through the micro-fluidic channel can also be identified as a step of translocating the strand through the micro-fluidic channel. The moving/translocating is performed longitudinally, i.e. the strand is moved along the longitudinal axis of the channel, from one end to the other end of the channel.

In an embodiment, each detector element may comprise two electrodes positioned longitudinally along the micro-fluidic channel and spanning the width of the detector element; and the detection signals are voltage changes between the two electrodes; and the micro-fluidic device further comprises a means for applying an ionic current through an electrolyte in the micro-fluidic channel. In this embodiment, the step of detecting the labels may comprise:
- applying an ionic current through the micro-fluidic channel using the means for applying an ionic current; and
- detecting the labels by detecting (and optionally measuring) a voltage change using the measurement unit.

In an embodiment, each detector element may comprise two electrodes facing each other on opposite sides of the micro-fluidic channel, each detector element being adapted for allowing the application of a DC bias superimposed to an AC voltage between the two electrodes and wherein the detection signals are impedance or capacitance changes. In this embodiment, the step of detecting the labels may comprise:
- detecting (and optionally measuring) a capacitance or impedance change using the measurement unit.

In an embodiment, the plurality of detector elements may be composed of:
a) a corresponding number of single electrodes, and
b) a single counter electrode common to each of the single electrodes, and the detection signal may be a double layer capacitance change . In this embodiment, the liquid may be an electrolyte solution and the electrolyte concentration of the solution may be tuned such that an electrical double layer at a surface of each detector element extends over at least half of the channel thickness in order to influence a double layer capacitance of the labels; and the detecting of the labels may comprise detecting (and optionally measuring) a double layer capacitance change using the measurement unit. In this measurement the impedance is measured by applying a DC bias voltage and a superimposed small AC voltage with a given frequency between the detector units and the counter electrode. Preferably, the frequency of the AC voltage for the impedance measurement should be sufficiently low, i.e. below the cut-off frequency of the ions.

In any embodiment of this aspect of the present invention, the method may further comprise prior to the step of providing the liquid in the micro-fluidic channel, a step of elongating the DNA or RNA strand in said micro-channel. For this purpose, means for elongating the DNA or RNA strand may be used.

In any embodiment of this aspect of the present invention, the method may further comprise prior to the step of providing the liquid in the micro-fluidic channel, a step of labeling a DNA or RNA strand at a plurality of specific sites with labels suitable for generating a detection signal when interacting with a detector element. In an embodiment, the labeling may comprises:
- providing a DNA or RNA strand;
- providing at least one DNA or RNA methyltransferase enzyme suitable for linking to a specific sequence of the DNA or RNA strand respectively;
- providing a co-factor for the enzyme, the co-factor being suitable for transferring a reactive group to each of the specific sequence via the methyltransferase enzyme;
- providing labels suitable for generating a detection signal when interacting with a detector element, the labels being functionalized to attach to the reactive group.

A methyltransferase catalyses the transfer of a methyl group from the ubiquitous cofactor s-adenosyl-L-methionine (SAM) to either a cytosine or adenine base, depending on the methyltransferase.

The methyltransferases are somewhat malleable. Their cofactor can be modified such that alkanes, aldehydes, ketones, aziridines and rather more extended propargyllic compounds (as used in the mTAG reaction) can be transferred in place of the natural methyl group (see scheme 1 below).

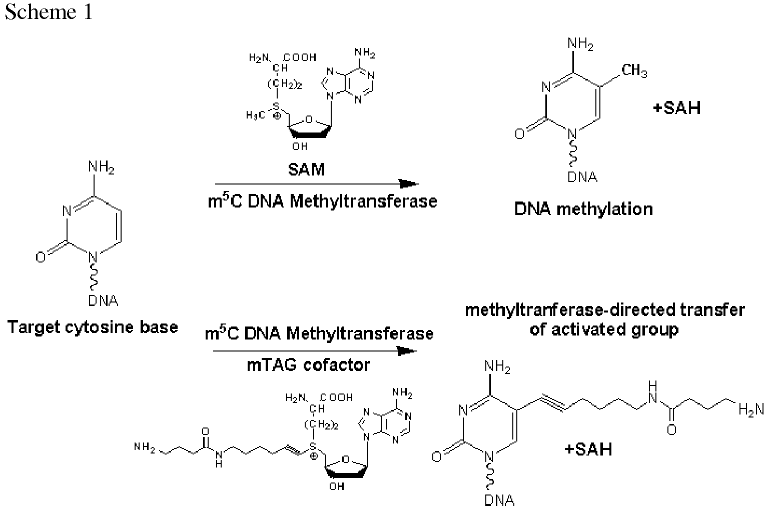

The above reaction scheme shows (top) the DNA methylation reaction and (bottom) the methyltransferase-directed transfer of activated groups. The product of the mTAG reaction is a DNA molecule that is 'activated', i.e. it carries a first reactive group (here an amine) suitable for reaction with e.g. a label bearing a second reactive group capable of reacting with the first reacting group (e.g. a carboxylic acid) at each target site for the DNA methyltransferase.

The specific sequence can for instance be from 3 to 10 bases in length, preferably from 4 to 6 bases in length.

In a further aspect, the present invention relates to a micro-fluidic chip comprising:
- a plurality of micro-fluidic devices according to any embodiment of the corresponding aspect of the present invention;
- a liquid supply unit connected to the plurality of micro-fluidic channels and arranged to provide a liquid comprising a plurality of DNA or RNA strands, each strand being labeled at a plurality of specific sites by labels suitable for generating a detection signal when interacting with a detector element, to the plurality of micro-fluidic channels. This aspect is advantageous as it permit a parallel format ensuring mapping with high speed.

In embodiments, the micro-fluidic chip may comprise a plurality of micro-fluidic devices according to any embodiment of the corresponding aspect of the present invention, each of said micro-fluidic devices comprising a measurement unit (or said micro-fluidic devices sharing a measurement unit), the micro-fluidic chip further comprising a computational unit connected to the measurement unit(s) and adapted for mapping the plurality of DNA or RNA strands by determining the distance between pairs of labels on each of the plurality of DNA or RNA strands using the timing information of the detected labels.

In yet a further aspect, the present invention relates to a method for performing multiplexed mapping of DNA or RNA strands using the micro-fluidic chip as described above, making use of a plurality of micro-fluidic devices for mapping a DNA or RNA strand labeled at a plurality of specific sites with labels suitable for generating a detection signal when interacting with a detector element, each device comprising:
- a micro-fluidic channel;
- a plurality of detector elements for detecting the labels by acquiring the detection signals, the detector elements being positioned longitudinally along the micro-fluidic channel, each detector element having a width, successive detector elements being separated by a inter-detector gap having a width, wherein the widths of at least two successive detector elements are different and/or wherein the width at least two of the inter-detector gaps are different;
- a measurement unit connected to the plurality of detector elements, the measurement unit being adapted for receiving and for timing the detection signals from each of the detector elements and optionally for measuring the magnitude of the detection signals from each of the detector elements;
- a means for moving the DNA or RNA strand through the micro-fluidic channel, the means being connected to the micro-fluidic channel. The method comprises:
- providing a liquid comprising a plurality of DNA or RNA strands, each being labeled at a plurality of specific sites by labels suitable for generating a detection signal when interacting with a detector element, to the fluid supply unit;
- moving each of the plurality of DNA or RNA strands through any of the plurality of micro-fluidic channels using one or more means for moving the DNA or RNA strands;
- detecting the labels of each of the plurality of DNA or RNA strands using the measurement units;
- mapping each of the plurality of DNA or RNA strands using the computational unit by determining the distance between pairs of labels on each of the plurality of DNA or RNA strands using the timing information of each of the detected labels.

In a further aspect, the present invention also includes a computer program or computer program product which, when executed on computing means, provides instructions for executing any of the methods of the present invention. Such computer program product can be tangibly embodied in a carrier medium carrying machine-readable code for execution by a programmable processor. The present invention thus also relates to a carrier medium carrying a computer program product that, when executed on computing means, provides instructions for executing any of the methods as described above. The term "carrier medium" refers to any medium that participates in providing instructions to a processor for execution. Such a medium may take many forms, including but not limited to, non-volatile media, and transmission media. Non volatile media includes, for example, optical or magnetic disks, such as a storage device which is part of mass storage. Common forms of computer readable media include, a CD-ROM, a DVD, a flexible disk or floppy disk, a memory key, a tape, a memory chip or cartridge or any other medium from which a computer can read. Various forms of computer readable media may be involved in carrying one or more sequences of one or more instructions to a processor for execution. The computer program or computer program product can be carried on an electrical carrier signal. The computer program product can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

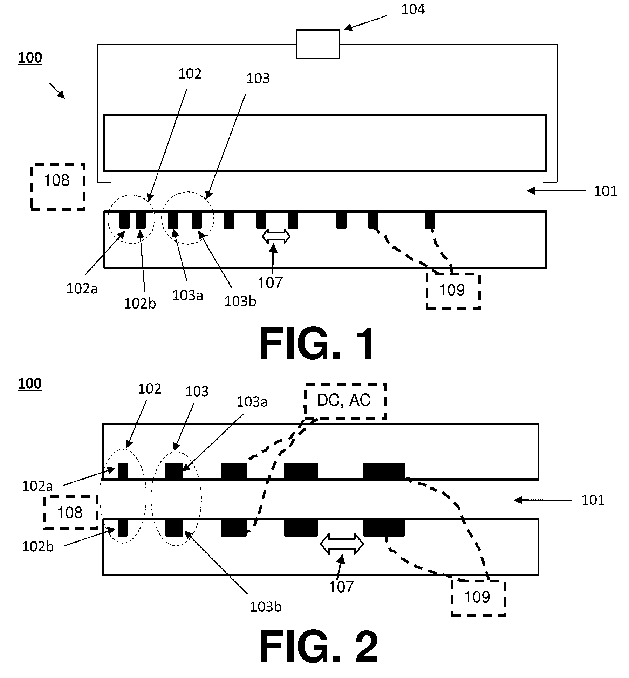

The following particular embodiments are illustrated in figures 1 to 4. For each of these embodiments, labeled DNA is first elongated in a nanochannel. These nanochannels contain detector elements (electrical contacts) that enable to detect the passing of the electrical labels. There are different read-out schemes possible. Some of them are described below:

### Example 1: Ionic current based read-out

In this embodiment (see figure 1) a constant ionic current is sent through the nanochannel (101), while the voltage change occurring upon the passing of a label (106) is measured between both contacts (102a, 102b) forming a detector element (e.g. detector element (102) composed of contact (102a) and contact (102b)). The DNA strand (105) is processed in a water based solution that contains a high (1 mM to 1M) concentration of electrolytes (salts, such as KaCl or NaCl) that generates a current flow upon the application of an external bias voltage (104). The nanochannel (101) can be considered as an ionic resistor. A constant current will result in a voltage drop across the nanochannel (101). The presence of objects (106) in the channel (101) results in a local reduction or increase of the ion concentration, yielding a locally varying resistance. Equipping the nanochannel (101) with an array of detector elements (102, 103) each composed of two electrodes (e.g. 102a and 102b) along the channel (101) allows probing the local variations of the resistance. Upon passage of a label (106), which can be a metallic or dielectric nanoparticle (106), the voltage drop changes, resulting in a time-dependent signal that can be translated to the positions along the DNA strand (105). The width (d) of the detector elements (102, 103), can be made small, as small as 50 nm and up to 200 nm wide. It is difficult to lithographically control the exact width of the detector elements (102, 103) with nanometer accuracy. This yields uncertainties on linking duration of events (time domain) to spatial information. However, it is much easier to lithographically produce structures composed of multiple detector elements (102, 103), wherein despite the fact that the size of one detector element (e.g. 102) cannot be exactly defined (e.g. we aim at 100 nm and we actually get 97 nm), the increment in size of a second element (e.g. 103) compared to the first element (102) can be controlled with nanometer accuracy (e.g. we aim for an increment of 10 nm and we actually get a first detector element (102) of width 97 nm, a second detector element (103) of width 107 nm, a third detector element of width 117 nm, etc). So, if the distance between the electrodes (102a, 102b) constituting the first detector element (102) is d, the distance between the next two electrodes (constituting the second detector element) can be d + I (increment), the distance between the n^{th} two electrodes (constituting the nth detector element) can be d + (n-1)I.

The increment I can for instance be taken as a value comprised between 1 and 50 nm. In the present example, the increment I is 10 nm.

The mapping, i.e. the determining of the distance between pairs of labels (106) on the DNA strand (105) can be performed as follow:
a) the time span (a) of a signal recorded when a label (106) flows over a detector element (102) of length d (e.g. 97 nm) is measured, and
b) the time span (b) of a signal recorded when this same label (106) flows over a subsequent detector (103) element of length d + increment i (e.g. d=97 nm + 10nm) is measured.

The difference between time span (b) and time span (a) gives us the time needed for the label (106) to flow over a distance of 10 nm. This gives us the speed of the DNA strand (105). Using more detector elements with d+20 nm, d+30 nm, etc. permits to obtain a better approximation of the speed.

Knowing the speed, we can determine the actual distance along the DNA strand (105) with accuracy in the order of one nanometer between any two labels (106) by multiplying the corresponding inter-signal time (the time between the detection of two labels (106) by a same detector element (102)) by the speed.

This embodiment gives better results if the speed of the strand (105) is constant. This is however typically the case. This method has the advantage of providing inter-label distances with high accuracy (at the nanometer level).

### Example 2: Impedance or capacitance based read-out:

In this embodiment (see figure 2) the detector elements (102, 103) are each composed of a pair of electrodes (102a, 102b) positioned at opposite sides of the nanochannel (101) and the capacitance or impedance between the two electrodes (102a, 102b) is continuously monitored. In this measurement method the impedance is measured by applying a DC bias voltage between the electrodes (102a, 102b) and a superimposed small AC voltage with a given frequency. The impedance is measured at that particular frequency. The impedance between these electrodes (102a, 102b) can generally be treated as a resistor with a capacitor in parallel. It is well known that the presence of dielectric or metallic objects will change the capacitance, and correspondingly the impedance. The frequency of the AC voltage can be adapted to increase the label (106) induced signal. Specifically the frequency dependent permittivity of both the label (106) and the environment can be used to determine the optimal frequency. In order to sense the labels (106), it is preferred to increase the frequency to above the cut-off frequency of the ions in the solution, such that the measured capacitance is not influenced by the ion diffusion capacitance. The passage of the labels (106) (e.g. nano-objects, such as dielectric or metallic particles) induces changes to the local impedance, leading to a time-dependent signal. Similarly to the previous case (see example 1), the exact width of the electrodes (102a, 102b) composing a detector element (102) cannot easily be realized with nanometer accuracy. However, here also, the width increment between two subsequent detector elements (102, 103) can be realized with much higher accuracy. In the present example, the increment I is 10 nm. The mapping can therefore be realized similarly to example 1.

### Example 3: Double layer capacitance based read-out

In this embodiment (see figure 3) the salt concentration of the solution is tuned such that the electrical double layer at the surface of the electrode extends over at least half of the channel (101) thickness or width. Upon passage of a charged label (106), the double layer capacitance is influenced and can be picked up by the electrodes (102a, 102b) composing the detector element (102). This measurement is similar to the measurement of example 2, with the difference that the counter electrode is the same for all electrodes (102, 103) along the channel and that the frequency of the impedance measurement should be sufficiently low, i.e. below the cut-off frequency of the ions. The width of the electrodes (102a, 102b, 103a, 103b) is also varied incrementally (like in example 2) in order to more accurately determine the positions of the labels (106) along the strand (105). In the present example, the increment I is 10 nm.

The mapping can therefore be realized similarly to example 1.

### Example 4: increasing the inter-detector gaps (107) between detector elements (102, 103) as an alternative to increasing the width of the detector elements (102, 103) themselves.

This example is an alternative embodiment applicable to the read-out methodologies of examples 1 to 3. In this embodiment, instead of using detector elements (102, 103) of varying width, it is the width of the inter-detector gap (107) between successive detector elements (102, 103) which is incremented for successive inter-detector gaps (107) along the micro-fluidic channel (101). So, if the width of the first inter-detector gap (107) is d', the width of the next inter-detector gap (107) can be d + I (increment), the width of the n^{th} inter-detector gap (107) can be d + (n-1)I. The increments do not however necessarily need to be the same all along the length of the channel (101).

In this embodiment, two different mapping methods can be used. The first method (not depicted) is similar to the method used in examples 1 to 3 and comprises a measure of the time necessary for a label (106) to cross a first inter-detector gap (107), measuring the time necessary for the same label (106) to cross a second inter-detector gap (not shown), larger than the first inter-detector gap (107) by a predefined increment, then subtracting the time necessary to cross the first inter-detector gap (107) from the time necessary to cross the second inter-detector gap (not shown), thereby obtaining the time necessary for crossing the increment. The length of the increment divided by the time for crossing it gives us the speed as also explained in examples 1 to 3.

A second mapping method is depicted in Figure 4. In this method, the simultaneity of two signals produced by two labels (106) on two different detectors (102, 103) is measured. When two signals are simultaneous (Figure 4 (a)), this means that the two labels are separated by the distance between the detector elements (102, 103). If exact simultaneity is not obtained, the distance between both labels can be approximated by extrapolation from the two pairs of signals where simultaneity was best approached. This embodiment works, but since it is very difficult to define exactly the distance between two detector elements (102, 103), the accuracy obtained by this embodiment is not as good as the accuracy obtained by the first mapping method. The accuracy is nevertheless better than the accuracy typically obtainable by optical methods. An advantage of this second mapping method is that it works equally well independently of the speed constancy of the strand (105).

### Example 5: Labeling of a DNA strand

20 µg of the DNA of a lambda bacteriophage (Fermentas) is modified using a methyltransferase M.Hhal (variant Q82A/Y254S/N304A) (equimolar amount of the target sites) and 20µM synthetic cofactor Ado-11-amino (see scheme 1) in 400 µl of M.Hhal buffer (50 mM Tris.HCl pH 7.4, 15 mM NaCl, 0.01 % 2-mercaptoethanol, 0.5 mM EDTA, 0.2 mg ml⁻¹ BSA) for 30 min at 37°C. The completion of the modification reaction is verified by treating a 10 µl aliquot with R. Hin6I (Fermentas) and agarose gel electrophoresis. The modified DNA is then incubated with 187 µg of Proteinase K (Fermentas) in the M.Hhal buffer supplemented with 0.025% SDS for Ih at 55 °C. DNA is purified by passing through a 1.6 ml Sephacryl^{™} S-400 column in PBS buffer followed by isopropanol precipitation. The pellet is dissolved in an appropriate solvent and incubated with an excess of gold nanoparticles bearing a reactive group capable of reacting with an amino group. The functionalized gold nanoparticles are carboxylic acid functionalized 15 nm gold nanoparticles obtained from Sigma-Aldrich. The resulting DNA strand is labeled at sequence specific sites (5'-GCGC-3') with gold nanoparticles.

The above-described method embodiments of the present invention may be implemented in a processing system (1) such as shown in Fig. 5. Fig. 5 shows one configuration of processing system (1) that includes at least one programmable processor (13) coupled to a memory subsystem (5) that includes at least one form of memory, e.g., RAM, ROM, and so forth. It is to be noted that the processor (13) or processors may be a general purpose, or a special purpose processor, and may be for inclusion in a device, e.g., a chip that has other components that perform other functions. Thus, one or more aspects of the present invention can be implemented in digital electronic circuitry, or in computer hardware, firmware, software, or in combinations of them. The processing system (1) may include a storage subsystem (12) that has at least one input port (e.g. disk drive and/or CD-ROM drive and/or DVD drive). In some implementations, a display system, a keyboard, and a pointing device may be included as part of a user interface subsystem (9) to provide for a user to manually input information. Ports for outputting data also may be included. More elements such as network connections, interfaces to various devices, and so forth, may be included, but are not illustrated in Fig. 5. The various elements of the processing system (1) may be coupled in various ways, including via a bus subsystem (11) shown in Fig. 5 for simplicity as a single bus, but will be understood to those in the art to include a system of at least one bus. The memory of the memory subsystem (5) may at some time hold part or all (in either case shown as (4)) of a set of instructions that when executed on the processing system (1) implement the steps of the method embodiments described herein. Thus, while a processing system (1) such as shown in Fig. 5 is prior art, a system that includes the instructions to implement aspects of the methods for mapping a DNA or RNA strand, or of the methods for performing multiplexed mapping of DNA or RNA strands is not prior art, and therefore Fig. 5 is not labelled as prior art.

### Figure 7 schematically represents a micro-fluidic chip (112) comprising:

- a plurality of micro-fluidic devices (100);
- a liquid supply unit (111) connected to the plurality of micro-fluidic channels (101) and arranged to provide a liquid comprising a plurality of DNA or RNA strands (105) to the plurality of micro-fluidic channels (101).

It is to be understood that although preferred embodiments, specific constructions and configurations, as well as materials, have been discussed herein for devices according to the present invention, various changes or modifications in form and detail may be made without departing from the scope of this invention. For example, steps may be added or deleted to methods described within the scope of the present invention.

## Claims

1. A micro-fluidic device (100) for mapping a DNA or RNA strand (105) labeled at a plurality of specific sites with labels (106) suitable for generating a detection signal when interacting with a detector element (102, 103), the device comprising:
- a micro-fluidic channel (101); and
- a plurality of detector elements (102, 103) for detecting the labels (106) by acquiring the detection signals, the detector elements (102, 103) being positioned longitudinally along the micro-fluidic channel (101), each detector element (102, 103) having a width, successive detector elements (102, 103) being separated by an inter-detector gap (107) having a width, wherein the widths of at least two of the detector elements (102, 103) are different and/or wherein the widths at least two of the inter-detector gaps (107) are different.

2. The micro-fluidic device (100) according to claim 1, further comprising a measurement unit (109) connected to the plurality of detector elements (102, 103), the measurement unit (109) being adapted for receiving and for timing the detection signals from each of the detector elements (102, 103) and optionally for measuring the magnitude of the detection signals from each of the detector elements (102, 103).

3. The micro-fluidic device (100) according to claim 1 or claim 2, further comprising a means (108) for moving the DNA or RNA strand (105) through the micro-fluidic channel (101), the means (108) being connected to the micro-fluidic channel (101).

4. The micro-fluidic device (100) according to any one of the preceding claims, wherein the device comprises at least three successive detector elements (102, 103) of increasing width along the micro-fluidic channel (101) and/or wherein the device (100) comprises at least three successive inter-detector gaps (107) of increasing width along the micro-fluidic channel (101).

5. The micro-fluidic device (100) according to any one of the preceding claims, wherein each detector element (102, 103) comprises two electrodes (102a, 102b; 103a, 103b) positioned longitudinally along the micro-fluidic channel (101), wherein both electrodes (102a, 102b; 103a, 103b) together span the width of the detector element (102, 103), and wherein the detection signals are voltage changes between the two electrodes (102a, 102b; 103a, 103b).

6. The micro-fluidic device (100) according to claim 5, further comprising a means for applying an ionic current (104) through an electrolyte in the micro-fluidic channel (101).

7. The micro-fluidic device (100) according to any one of claims 1 to 4 wherein each detector element (102, 103) comprises two electrodes (102a, 102b; 103a, 103b) facing each other on opposite sides of the micro-fluidic channel (101), the detector element (102, 103) being adapted for allowing the application of a DC bias superimposed to an AC voltage between the two electrodes (102a, 102b; 103a, 103b) and wherein the detection signals are impedance or capacitance changes.

8. The micro-fluidic device (100) according to any one of claims 1 to 4 wherein the plurality of detector elements is composed of:
a) a corresponding number of single electrodes (102, 103), and
b) a single counter electrode (110) common to each of the single electrodes (102, 103), and wherein the detection signal is a double layer capacitance change.

9. A method for mapping a DNA or RNA strand (105) using the micro-fluidic device (100) according to claim 3 as depending on claim 2, the method comprises the steps of:
- providing a liquid comprising a DNA or RNA strand (105) in the micro-fluidic channel (101), the strand (105) being labeled at a plurality of specific sites by labels (106) suitable for generating a detection signal when interacting with a detector element (108);
- moving the strand (105) through the micro-fluidic channel (101);
- detecting the labels (106) using the measurement unit (109) and recording timing information of the detected labels (106); and
- mapping the DNA strand (105) by using the timing information of the detected labels (106) for determining the distance between pairs of labels (106), thereby determining the distance between the specific sites.

10. The method according to claim 9 using the micro-fluidic device (100) according to claim 6 wherein the detecting of the labels (106) comprises:
- applying an ionic current through the micro-fluidic channel (101) using the means for applying an ionic current (104); and
- detecting the labels (106) by detecting a voltage change using the measurement unit (109).

11. The method according to claim 9 using the microfluidic device (100) according to claim 7 wherein the detecting of the labels (106) comprises:
- detecting a capacitance or impedance change using the measurement unit (109).

12. The method according to claim 9, using the micro-fluidic device (100) according to claim 8, wherein the liquid is an electrolyte solution and wherein the electrolyte concentration of the solution is tuned such that an electrical double layer at a surface of each detector element (102, 103) extends over at least half of the channel (101) thickness in order to influence a double layer capacitance of the labels (106); and wherein the detecting of the labels (106) comprises detecting a double layer capacitance change using the measurement unit (109).

13. A micro-fluidic chip (112) comprising:
- a plurality of micro-fluidic devices (100) according to claim 1 to 8;
- a liquid supply unit (111) connected to the plurality of micro-fluidic channels (101) and arranged to provide a liquid comprising a plurality of DNA or RNA strands (105) to the plurality of micro-fluidic channels (101).

14. A method for performing multiplexed mapping of DNA or RNA strands (105) using the micro-fluidic chip (112) according to claim 13, making use of a plurality of micro-fluidic devices (100) according to claim 3 as depending on claim 2, the method comprising:
- providing a liquid comprising a plurality of DNA or RNA strands (105), each being labelled at a plurality of specific sites by labels (106) suitable for generating a detection signal when interacting with a detector element (108), to the fluid supply unit (111);
- transferring each of the plurality of DNA or RNA strands (105) to any of the plurality of micro-fluidic channels (101),
- moving each of the plurality of DNA or RNA strands (105) through the micro-fluidic channels (101) to which it has been transferred, using one or more means (108) for moving the DNA or RNA strands (105) ;
- detecting the labels (106) of each of the plurality of DNA or RNA strands (105) using the measurement units (109);
- mapping each of the plurality of DNA or RNA strands (105) by determining the distance between pairs of labels (106) on each of the plurality of DNA or RNA strands (105) using the timing information of each of the detected labels (106).

15. A computer program product that, when executed on computing means (1), provides instructions for executing any one of methods 9 to 12 and 14.
